# FASCICULE DE BREVET EUROPEEN

(11) **EP 1 907 396 B1**
(45) Date de publication et mention de la délivrance du brevet: **19.11.2008**
(21) Numéro de dépôt: 06764221.5
(22) Date de dépôt: 19.07.2006
(51) Int. Cl.: C07D 493/04

(54) **PROCEDE DE PREPARATION DE LA 4 -AMINO-4'-DEMETHYL-4-DESOXYPODOPHYLLOTOXINE**
VERFAHREN ZUR HERSTELLUNG VON 4-AMINO-4'-DEMETHYL-4-DESOXYPODOPHYLOTOXIN
METHOD FOR PREPARING 4 -AMINO-4'-DEMETHYL-4-DESOXYPODOPHYLLOTOXIN

(30) Priorité: 19.07.2005 FR 0507642
(43) Date de publication de la demande: 09.04.2008
(73) Titulaire: PIERRE FABRE MEDICAMENT, 92100 Boulogne-Billancourt (FR)
(72) Inventeur: GUMINSKI, Yves, F-81090 Lagarrigue (FR); GROUSSEAUD, Martial, F-81100 Castres (FR); IMBERT, Thierry, F-81290 Viviers Les Montagnes (FR)
(74) Mandataire: Ahner, Francis
(86) Numéro de dépôt international: PCT/EP2006/064420
(87) Numéro de publication internationale: WO 2007/010007

(56) Documents cités:
- KAMAL A ET AL: "Synthesis of 4-beta-Amido and 4-beta-Sulphonamido Analogues of Podophyllotoxin as Potential Antitumor Agents" BIOORGANIC & MEDICINAL CHEMISTRY, ELSEVIER SCIENCE LTD, GB, vol. 11, no. 23, 17 novembre 2003 (2003-11-17), pages 5135-5142, XP002281030 ISSN: 0968-0896 cité dans la demande
- MASAKI M ET AL: "A new method for the removal of chloroacetyl groups" JOURNAL OF THE AMERICAN CHEMICAL SOCIETY, vol. 90, 31 juillet 1968 (1968-07-31), pages 4508-4509, XP002397159
- JIRGENSONS A ET AL: "A practical synthesis of tert-alkylamines via the ritter reaction with chloroacetonitrile" SYNTHESIS, GEORG THIEME VERLAG, STUTTGART, DE, no. 12, 2000, pages 1709-1712, XP002375279 ISSN: 0039-7881 cité dans la demande

## Description

La présente invention concerne un procédé de préparation de la 4β-amine-4'-déméthyl-4-desoxypodophyllotoxine de formule 1, à partir la 4β-halogenoacetamido-4'-déméthyl-4desoxypodophyllotoxine de formule 3 (X = Cl, Br ou I), par clivage en présence de thiourée et d'un acide. En particulier, la présente invention concerne un procédé de préparation de la 4β-amino-4'-déméthyl-4-desoxypodophyllotoxine de formule 1, à partir de la 4'-déméthylepipodophyllotoxine de formule 2, via la 4β-halogenoacetamido-4'-déméthyl-4-desoxypodophyllotoxine de formule 3 (X = Cl, Br ou I).

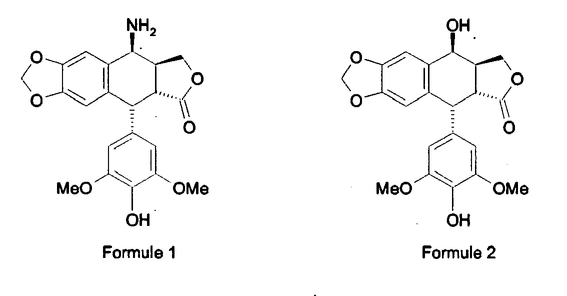

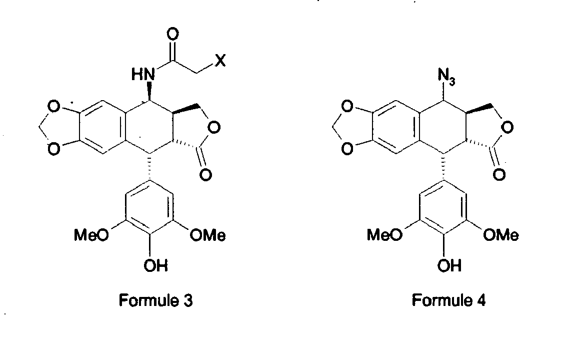

La 4β-amino-4'-déméthyl-4-desoxypodophyllotoxine est un intermédiaire de synthèse utile dans la préparation de composés anticancéreux (demande de brevet français n° 0404053).

La stratégie de la préparation de cet intermédiaire repose sur la transformation de la 4'-déméthylépipodophyllotoxine (formule 2) en 4β-azido-4'-déméthyl-4-desoxypodophyllotoxine de formule 4, puis par réduction catalytique de ce dérivé azide en dérivé amino de formule 1. Le problème de cette transformation est le manque de stéréosélectivité de la transformation du dérivé activé en position 4 (position benzylique), fournissant le mélange des azides α et β de formule 4. Ce problème a été en partie résolu dans J.Med.Chem. 1991, 34, 3346, en utilisant l'azoture de sodium et l'acide trifluoroacétique. Il était nécessaire cependant de purifier l'intermédiaire azido de formule 4, par chromatographie, ainsi que le produit de réduction catalytique, c'est à dire l'amino de formule 1. Un autre procédé a été décrit dans Chinese Chemical Letters 1993, 4 (4) 289. Ces auteurs utilisent la méthode à l'azide, mais en faisant réagir l'acide azothydrique HN₃ (préparé in situ), en présence d'éthérate de BF₃ à -10 ~ -15°C. Les résultats de ces auteurs indiquent une bonne stéréoselectivité de la transformation, avec un rendement au moins égal à 80%. Un procédé de transformation de l'azido de formule 4 en amino de formule 1 est également décrit dans Tet.Let. 1999, 40, 1967 et Tet.Let. 2000, 41, 7743. Ces auteurs utilisent l'iodure de Samarium dans le t-BuOH et le THF, ou bien le couple FeSO₄.7H₂O/NH₃. Récemment, Bioorg.Med.Chem. 2003, 11, 5135 confirme la nécessaire purification chromatographique. Ils obtiennent l'amino de formule 1 avec un rendement de 70%.

Ces méthodes posent malgré tout deux problèmes. 1) l'utilisation d'un dérivé azide dangereux, potentiellement explosif, surtout lors d'une utilisation à grande échelle pour la préparation industrielle d'un médicament et 2) le nécessaire passage par une ou même 2 étapes de chromatographie pour fournir un composé amino de formule 1 de bonne qualité pour préparer ultérieurement le produit fini, médicament anticancéreux, qui représentent des étapes onéreuses sur le plan industriel.

L'objet de la présente invention est la résolution de ces deux problèmes, en n'utilisant pas de composés dangereux ou explosifs, et en n'ayant pas besoin d'étapes de purification chromatographique.

La 4β-halogénoacétamido-4'-déméthyl-4-desoxypodophyllotoxine, intermédiaire de formule 3, est un composé déjà connu (demande de brevet français n° 0404053, WO2004/073375). De même, le passage du composé de formule 2 au composé de formule 3 est déjà connu (demande de brevet français n° 0404053). L'objet de l'invention est donc un procédé de synthèse du composé de formule 1 à partir du composé de formule 3.

En synthèse organique classique, le clivage des chloracétamides pour obtenir des amines est réalisé par traitement d'un dérivé chloracétamido d'amide tertiaire par la thiourée dans l'éthanol en présence d'acide acétique, dans une proportion optimale de 5 :1 (A. Jirgensons et al., Synthesis 2000, 1709). Dans cette réaction, l'éthanol et l'acide acétique utilisés ne contiennent pas d'eau. Cette méthode n'a jamais été appliquée en série podophyllotoxine et elle ne convient pas. En effet, appliquée au cas du composé de formule 3, la méthode enseignée conduit après 10 heures sous reflux à une transformation de moins de 10% de la matière première engagée (composé de formule 3), et l'intermédiaire réactionnel (X = S-isothiouronium sous forme de chlorure) ne réagit plus (cf exemple comparatif). Un temps plus long de réaction est défavorable en terme de pureté, avec l'apparition de produits secondaires.

Il a fallu adapter et améliorer le mode opératoire pour obtenir la transformation voulue. D'une manière surprenante, les inventeurs ont déterminé un procédé de synthèse du composé de formule 1, à partir du composé de formule 3, qui permet l'obtention du composé de formule 1 avec une bonne pureté, sans étape supplémentaire de purification (chromatographie en particulier).

La présente invention a donc pour objet un procédé de synthèse de la 4β-amino-4'-déméthyl-4-desoxypodophyllotoxine de formule 1 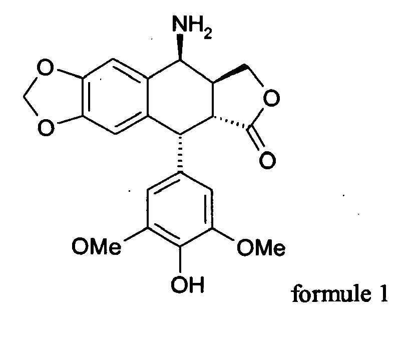 caractérisé en ce qu'il comprend les étapes successives suivantes :
a) réaction, dans un acide faible pur, sans autre solvant, à une température supérieure à la température ambiante, de la thiourée avec la 4β-halogénoacétamido-4'-déméthyl-4-desoxypodophyllotoxine de formule 3 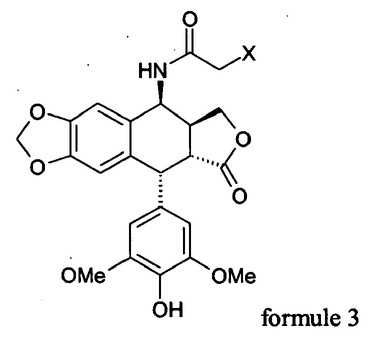 dans laquelle X représente un atome d'halogène choisi dans le groupe constitué par le chlore, le brome et l'iode, avantageusement le chlore ;
b) récupération de la 4β-amino-4'-déméthyl-4-desoxypodophyllotoxine.

Par rapport au procédé de clivage des chloracétamides décrit dans l'art antérieur (A. Jirgensons et al., Synthesis 2000, 1709), il a été trouvé que l'opération peut se conduire dans un acide faible pur, c'est-à-dire sans eau ou autre solvant organique. Au sens de la présente invention, l'emploi du terme « acide » fait référence à la définition de Bronsted, à savoir une espèce chimique capable de céder un proton H⁺. Un acide faible est un acide qui ne se dissocie pas totalement dans l'eau, contrairement à un acide fort.

L'acide faible a avantageusement une valeur de pKa comprise entre 4 et 6 à 25°C. En particulier, l'acide faible est avantageusement un acide carboxylique de formule 5 R-COOH, dans laquelle R représente un hydrogène ou un radical alkyle en C₁-C₂. Les acides plus lourd ne sont plus utilisables comme solvant, ou n'ont pas les caractéristiques olfactives convenables (l'acide butyrique en particulier). Plus particulièrement, l'acide faible est choisi dans le groupe constitué par l'acide formique, l'acide acétique ou l'acide propionique, de préférence l'acide acétique. Dans ce qui suit, les proportions données entre les différents composés correspondent aux proportions de quantités engagées pour ces composés, sauf indication contraire.

Dans le cadre de la présente invention, l'expression « acide faible pur » signifie que cet acide est glacial, c'est-à-dire exempt d'eau. L'expression « sans autre solvant » signifie que le milieu réactionnel de l'étape a) ne comprend que l'acide faible pur, le composé de formule 3 et la thiourée, ainsi il ne comprend pas d'eau ou un autre solvant, tel qu'un alcool ou un solvant organique.

Lors de l'étape a), le milieu réactionnel est avantageusement chauffé à une température supérieure à 60°C, plus avantageusement comprise entre 60 et 100°C. Une autre caractéristique de l'invention tient en ce que l'acide faible pur utilisé sert de solvant à la réaction. Le rapport molaire entre la 4β-halogénoacétamido-4'-déméthyl-4-desoxypodophyllotoxine et l'acide faible est au moins de 0,5.

Le rapport molaire entre la 4β-halogénoacétamido-4'-déméthyl-4-desoxypodophyllotoxine et la thiourée est avantageusement compris entre 0,5 et 1. Selon une variante avantageuse de l'invention, lors de l'étape a), la 4β-halogénoacétamido-4'-déméthyl-4-desoxypodophyllotoxine est mise en contact avec l'acide faible pur avant ajout de la thiourée. Selon une variante encore plus avantageuse, la 4β-halogénoacétamido-4'-déméthyl-4-desoxypodophyllotoxine et l'acide faible pur sont mis en contact, la 4β-halogénoacétamido-4'-déméthyl-4-desoxypodophyllotoxine étant avantageusement en suspension dans l'acide faible pur, et le milieu réactionnel est chauffé jusqu'à la température désirée avant ajout de la thiourée à cette température.

Le temps de réaction de l'étape a) est avantageusement compris entre 1 et 3 heures. Dans le cas de l'acide acétique pur, le temps de réaction de l'étape a) est d'environ 2 heures.

Suite à l'étape a), le produit final de formule 1 précipite dans le milieu réactionnel. Il est récupéré lors de l'étape b) par toute technique connue de l'homme du métier, en particulier une simple filtration et un séchage selon les méthodes usuelles suffisent.

Une fois filtré et séché selon les méthodes usuelles, le composé de formule 1, sous forme de chlorhydrate, bromhydrate ou iodhydrate, est obtenu avec un rendement molaire moyen supérieur à 85%, avantageusement supérieur à 90% basé sur la quantité molaire de composé de formule 3 engagé. Dans le cas de l'acide acétique pur, le composé de formule 1, sous forme de chlorhydrate, bromhydrate ou iodhydrate, est obtenu avec un rendement molaire moyen de 93% basé sur la quantité molaire de composé de formule 3 engagé.

Le composé de formule 1 est avantageusement obtenu avec un degré de pureté supérieur à 90%, plus avantageusement supérieur ou égal à 95%.

Le composé de formule 1, obtenu sous forme de chlorhydrate, bromhydrate ou iodhydrate, est pur et ne nécessite aucune étape supplémentaire de purification chromatographique. Il peut être utilisé directement pour des étapes ultérieures de synthèse, ce qui représente un avantage important au point de vue préparatif sur le plan économique et industriel.

La présente invention a également pour objet un procédé de synthèse de la 4β-amino-4'-déméthyl-4-desoxypodophyllotoxine de formule 1 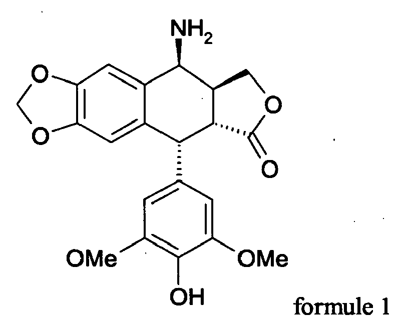 caractérisé en ce qu'il comprend les étapes successives suivantes :
i) réaction, dans un mélange d'acide, d'eau et de solvant organique, à une température supérieure à la température ambiante, de la thiourée avec la 4β-halogénoacétamido-4'-déméthyl-4-desoxydophyllotoxine de formule 3 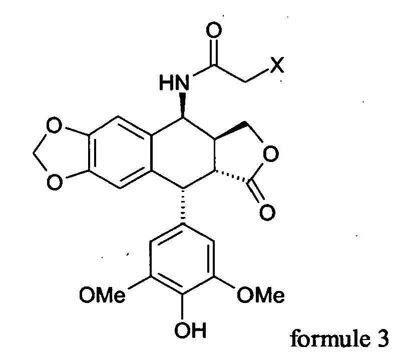 dans laquelle X représente un atome d'halogène choisi dans le groupe constitué par le chlore, le brome et l'iode, avantageusement le chlore ;
ii) récupération de la 4β-amino-4'-déméthyl-4-desoxypodophyllotoxine. Par rapport au procédé de clivage des chloracétamides décrit dans l'art antérieur (A. Jirgensons et al., Synthesis 2000, 1709), il a été trouvé que l'addition d'eau au milieu réactionnel favorisait la réaction en consommant complètement la matière première, sans apparition de produits de dégradation.

Le milieu réactionnel ne contient avantageusement pas d'autre solvant ou réactif. Lors de l'étape i), le milieu réactionnel est avantageusement chauffé à une température supérieure à 60°C, plus avantageusement comprise entre 60 et 100°C. Le rapport molaire entre la 4β-halogénoacétamido-4'-déméthyl-4-desoxypodophyllotoxine et la thiourée est avantageusement compris entre 0,5 et 1. Lors de l'étape i), la 4β-halogénoacétamido-4'-déméthyl-4-desoxypodophyllotoxine est avantageusement mise en contact avec le mélange d'acide, d'eau et de solvant organique avant ajout de la thiourée. D'une manière encore plus avantageuse, la 4β-halogénoacétamido-4'-déméthyl-4-desoxypodophyllotoxine et le mélange d'acide, d'eau et de solvant organique sont mis en contact, la 4β-halogénoacétamido-4'-déméthyl-4-desoxypodophyllotoxine étant avantageusement en suspension dans ledit mélange, et le milieu réactionnel est chauffé jusqu'à la température désirée avant ajout de la thiourée à cette température.

Le solvant organique utilisé dans le deuxième procédé selon l'invention est avantageusement un solvant organique hydrosoluble, plus avantageusement choisi dans le groupe constitué par les éthers cycliques, en particulier le dioxane, les alcools, en particulier le méthanol, l'éthanol, le propanol et l'isopropanol, et le N,N-diméthylacétamide (DMA), le diméthylformamide (DMF) et la N-méthylpyrrolidone (NMP).

Ainsi, par rapport au procédé de clivage des chloracétamides décrit dans l'art antérieur 1 (A. Jirgensons et al., Synthesis 2000, 1709), il a également été trouvé que l'opération peut se conduire en présence d'un solvant organique, comme le dioxane ou le DMA, en lieu et place de l'éthanol, en présence d'eau.

Selon une première variante avantageuse du deuxième procédé selon l'invention, le solvant organique est un alcool, avantageusement l'éthanol.

Dans le cadre de cette première variante, l'acide est avantageusement un acide fort, en particulier choisi dans le groupe constitué par l'acide chlorhydrique, l'acide sulfurique et l'acide phosphorique. Le rapport volumétrique alcool / (eau + acide fort) est avantageusement de 2 à 5 / 0,5 à 2, plus avantageusement de 2,5/1, l'acide fort étant une à deux fois normal (normalité comprise entre 1 et 2). Le composé de formule 1 est alors avantageusement obtenu avec un rendement molaire supérieur à 80%, plus avantageusement supérieur à 85%, avantageusement égal à 90%. Le temps de réaction est avantageusement supérieur à 8 heures, mais inférieur à 10 heures, encore plus avantageusement d'environ 9 heures.

Le composé de formule 1 est également avantageusement obtenu avec un degré de pureté supérieur à 90%, avantageusement de 95%.

Alternativement, dans le cadre de cette première variante, l'acide est avantageusement un acide faible, en particulier un acide carboxylique de formule 5 R-COOH, dans laquelle R représente un hydrogène ou un radical alkyle en C₁-C₂. Les acides plus lourds ne sont plus utilisables comme solvant, ou n'ont pas les caractéristiques olfactives convenables (l'acide butyrique en particulier). Plus particulièrement, l'acide faible est choisi dans le groupe constitué par l'acide formique, l'acide acétique ou l'acide propionique, de préférence l'acide acétique. Le rapport volumétrique alcool / eau / acide faible est avantageusement de 2 à 10 / 0,5 à 2 / 0,5 à 2, plus particulièrement de 5/1/1. Plus particulièrement, le rapport volumétrique éthanol / eau / acide acétique est avantageusement de 5/1/1.

Le composé de formule 1 est alors avantageusement obtenu avec un rendement molaire supérieur à 55%, avantageusement égal à 60%. Le temps de réaction est avantageusement supérieur à 8 heures, mais inférieur à 11 heures, encore plus avantageusement d'environ 10 heures.

Le composé de formule 1 est avantageusement obtenu avec un degré de pureté supérieur à 90%, avantageusement de 95%.

Selon une deuxième variante avantageuse du deuxième procédé selon l'invention, le solvant organique est un éther cyclique, en particulier le dioxane, et le DMA, le DMF ou la NMP.

Le rapport volumétrique éther cyclique (dioxane) ou DMA, DMF, NMP / eau / acide faible (acide acétique) est avantageusement de 2 à 10 / 0,5 à 2 / 0,5 à 2, plus particulièrement de 5/1/1. Le rapport volumétrique dioxane ou DMA, DMF, NMP / eau / acide acétique est avantageusement de 5/1/1. Le composé de formule 1 est alors avantageusement obtenu avec un rendement molaire supérieur à 60%, plus avantageusement supérieur à 65%, avantageusement égal à 70%. Le temps de réaction est avantageusement supérieur à 4 heures, mais inférieur à 10 heures, encore plus avantageusement d'environ 5-6 heures.

Le composé de formule 1 est alors avantageusement obtenu avec un degré de pureté supérieur à 90%, avantageusement de 95%.

Dans les cas étudiés, le produit final précipite dans le milieu réactionnel. Il est récupéré lors de l'étape b) par toute technique connue de l'homme du métier, en particulier une simple filtration et un séchage selon les méthodes usuelles suffisent.

Le composé de formule 1 obtenu, sous forme de chlorhydrate, bromhydrate ou iodhydrate, est pur et ne nécessite aucune étape supplémentaire de purification chromatographique. Il peut être utilisé directement pour des étapes ultérieures de synthèse, ce qui représente un avantage important au point de vue préparatif sur le plan économique et industriel.

Dans le cadre du premier ou du second procédé selon l'invention, la 4β-halogénoacétamido-4'-déméthyl-4-desoxypodophyllotoxine de formule 3 est avantageusement obtenue par réaction de la 4'-déméthylepipodophyllotoxine de formule 2 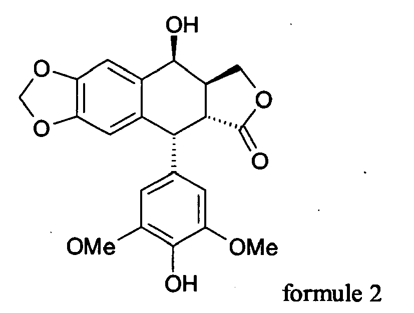 avec un halogénoacétonitrile de formule 6 X-CH₂-C≡N, dans laquelle X représente un atome d'halogène choisi dans le groupe constitué par le chlore, le brome et l'iode, en milieu acide. Il s'ensuit une réaction de Ritter pour fournir directement par cristallisation en fin de réaction le 4β-chloroacétamido-4'-déméthyl-4-desoxypodophyllotoxine avec un rendement avantageusement supérieur à 80%, encore plus avantageusement supérieur à 90%.

Cet intermédiaire possède exclusivement la stéréochimie β sur le carbone en position 4. Le problème de la stéréochimie est résolu à ce stade. La pureté de cet intermédiaire est telle qu'il peut être utilisé sans purification ultérieure dans l'étape de clivage pour fournir la 4β-amino-4'-déméthyl-4-desoxypodophyllotoxine de formule 1.

La 4'-déméthylepipodophyllotoxine de formule 2 (préparée selon le procédé décrit dans le brevet FR 2 742 439) est avantageusement traitée par le chloroacétonitrile, réactif usuel et bon marché, avec l'acide sulfurique. Le 4β-chloroacétamido-4'-déméthyl-4-desoxydophyllotoxine est alors avantageusement obtenu avec un rendement de 93%.

Une fois filtré et séché selon les méthodes usuelles, le composé de formule 1, sous forme de chlorhydrate, bromhydrate ou iodhydrate, est obtenu, dans le cadre de l'utilisation de l'acétique acétique glacial pur (ni eau ni autre solvant organique, premier procédé selon l'invention) avec un rendement molaire moyen de 86% basé sur la quantité molaire de 4'-déméthylepipodophyllotoxine (formule 2) engagée, c'est à dire sur 2 étapes (du composé de formule 2 au composé de formule 3 puis du composé de formule 3 au composé de formule 1).

Les exemples suivant montrent les techniques opératoires utilisées.

### Exemple 1: Préparation du 4β-chloroacétamido-4'-déméthyl-4-desoxypodophyllotoxine (formule 3).

A la suspension de 30g (0,075 mole) de 4'-déméthylépipodophyllotoxine dans 47,5 mL (0,75 mole) de chloroacétonitrile, on ajoute goutte à goutte 0,5 mL d'acide sulfurique concentré à température ambiante. On laisse agiter à cette température pendant 1 heure durant laquelle on observe une dissolution puis une reprécipitation. On additionne 300 mL de 2-propanol. Le précipité est filtré, rincé avec 200 mL de propanol-2 et de l'eau jusqu'à retour à pH 7. Le solide blanc obtenu est séché sous vide à 40°C pour donner 32,9 g du composé chloroacétamido de formule 3, soit un rendement molaire de 93%.
Point de fusion F = 240°C.
Analyse RMN du proton : ¹H RMN (DMSO) δ 8,65(d, 1H, J = 7 Hz, NH), 8,26(s, 1H, 4'-OH), 6,78(s, 1H, H₅), 6,54(s, 1H, H₈), 6,24(s, 2H, H_{2'}, H₆), 5,99 (d, 2H, J = 11.3 Hz, OCH₂O) 5,17(dd, 1H, J = 4,56 et 7 Hz, H₄), 4,51(d, 1H, J = 5,2 Hz, H₁), 4,29(t, 1H, J = 8 Hz, H₁₁ₐ), 4,10(s, 2H, CH₂Cl), 3,97(m, 1H, H₃), 3,78(dd, 1H, J = 8 Hz et 10 Hz, H_{11b}), 3,63(s, 6H, 2xOCH₃), 3,15(dd, 1H, J = 5,2 et 14 Hz, H₂).
Les autres halogénoacétamides (X = Br, I) s'obtiennent de façon analogue en utilisant le bromoacétonitrile ou le iodoacétonitrile.

### Exemple comparatif : Préparation de la 4-amino-4'-déméthyl-4-desoxypodophyllotoxine (formule 1) - Méthode avec éthanol : acide acétique 5 :1 (selon Synthesis 2000, 1709)

### Tableau 1 : Entrée 1.

Une suspension de 0,5g (1,05 mmoles) de 4β-chloroacétamido-4'-déméthyl-4-desoxypodophyllotoxine obtenu à l'exemple 1, dans un mélange de 2,5 mL d'éthanol et de 0,5 mL d'acide acétique glacial est porté à 80°C sous agitation. On ajoute en une fois 0,12 g (1,57 mmoles) de thiourée. On laisse agiter à cette température pendant 10 heures. L'analyse du milieu réactionnel, estimé par chromatographie en couche mince, ne révèle que moins de 10% du produit 4β-amino-4'-déméthylépipodophyllotoxine recherché (formule 1), de la présence de l'intermédiaire isothiouronium (X = S-isothiouronium) qui ne réagit plus ainsi que des produits de dégradation.

### Exemple 2 : Préparation de la 4β-amino-4'-déméthyl-4-desoxypodophyllotoxine (formule 1) - Méthode avec acide acétique glacial pur - premier procédé selon l'invention.

### Tableau 1 : Entrée 2.

Une suspension de 17g (0,0358 mole) de 4β-chloroacétamido-4'-déméthyl-4-desoxypodophyllotoxine obtenu à l'exemple 1, dans 75 mL d'acide acétique glacial est porté à 80°C sous agitation. On ajoute en une fois 4,2 g (0,0537 mole) de thiourée. On laisse agiter à cette température pendant 1 h 30, durant laquelle on observe une dissolution puis une reprécipitation. Le milieu réactionnel est filtré à chaud, rincé avec 75 mL d'acide acétique glacial et de l'éther diisopropylique. Le solide blanc obtenu est séché sous vide à 40°C pour donner 14,6g du composé de formule 1 sous sa forme chlorhydrate correspondant à un rendement molaire de 93%.
Point de fusion : F >260°C.
Analyse RMN du proton : ¹H RMN (DMSO) δ 8,63(m, 2H), 8,32(m, 1H), 7,23(s, 1H, H₅), 6,60 (s, 1H, H₈), 6,18 (s, 2H, H_{2'}, H_{6'}), 6,05 (d, 2H, J = 2,1 Hz, OCH₂O) 4,73 (d, 1H, J = 4,5 Hz, H₄), 4,56 (d, 1H, J = 5,2 Hz, H₁), 4,34 (m, 2H, H₁₁ₐ et H_{11b}), 3,65 (dd, 1H, J = 5,2 Hz, H₂), 3,62 (s, 6H, 2xOCH₃), 3,06 (m, 1H, H₃).

### Exemple 3 : Préparation de la 4β-amino-4'-déméthyl-4-desoxypodophyllotoxine (formule 1) - Méthode avec éthanol et acide chlorydrique 1N- second procédé selon l'invention, 1^{ère} variante, première alternative.

### Tableau 1 : Entrée 3.

Une suspension de 0,5g (1,05 mmoles) de 4β-chloroacétamido-4'-déméthyl-4-desoxypodophyllotoxine obtenu à l'exemple 1, dans un mélange de 2,5 mL d'éthanol et de 1 mL d'acide chlorhydique 1N est porté à 80°C sous agitation. On ajoute en une fois 0,12 g (1,57 mmoles) de thiourée. On laisse agiter à cette température pendant 9 heures durant laquelle on observe une dissolution puis une reprécipitation. Le milieu réactionnel refroidi est filtré, rincé avec de l'éthanol et de l'éther diisopropylique. Le solide blanc obtenu est séché sous vide à 40°C pour donner 0,4 g du composé de formule 1 sous sa forme chlorhydrate correspondant à un rendement molaire de 90%.
Point de fusion : F >260°C

### Exemple 4 : Préparation de la 4β-amino-4'-déméthyl-4-desoxypodophyllotoxine (formule 1) - Méthode avec éthanol : eau : acide acétique (5:1:1) - second procédé selon l'invention, 1^{ère} variante, deuxième alternative.

### Tableau 1 : Entrée 4.

Une suspension de 0,5g (1,05 mmoles) de 4β-chloroacétamido-4'-déméthyl-4-desoxypodophyllotoxine obtenu à l'exemple 1, dans un mélange de 2,5 mL d'éthanol, 0,5 mL d'eau et de 0,5 mL d'acide acétique glacial est porté à 80°C sous agitation. On ajoute en une fois 0,12 g (1,57 mmoles) de thiourée. On laisse agiter à cette température pendant 10 heures durant laquelle on observe une dissolution puis une reprécipitation. Le milieu réactionnel refroidi est filtré, rincé avec de l'éthanol et de l'éther diisopropylique. Le solide blanc obtenu est séché sous vide à 40°C pour donner 0,27 g du composé de formule 1 sous sa forme chlorhydrate correspondant à un rendement molaire de 60%.
Point de fusion : F >260°C.

### Exemple 5 : Préparation de la 4β-amino-4'-déméthyl-4-desoxypodophyllotoxine (formule, 1) - Méthode avec solvant (DMA; dioxane) / eau / acide acétique - second procédé selon l'invention, 2^{ème} variante.

### Tableau 1 : Entrée 5.

Une suspension de 0,5g (1,05 mmoles) de 4β-chloroacétamido-4'-déméthyl-4-desoxypodophyllotoxine obtenu à l'exemple 1, dans un mélange de 2,5 mL de dioxane ou de DMA, 0,5 mL d'eau et de 0,5 mL d'acide acétique glacial est porté à 80°C sous agitation. On ajoute en une fois 0,12 g (1,57 mmoles) de thiourée. On laisse agiter à cette température pendant 5 à 6 heures durant laquelle on observe une dissolution puis une reprécipitation. Le milieu réactionnel refroidi est filtré, rincé avec du 2-propanol et de l'éther diisopropylique. Le solide blanc obtenu est séché sous vide à 40°C pour donner 0,31 g du composé de formule 1 sous sa forme chlorhydrate correspondant à un rendement molaire de 70%.
Point de fusion : F >260°C

Les résultats des essais de l'exemple comparatif et des exemples 2 à 5 sont rassemblés dans le tableau 1 suivant :

| Entrée | Conditions | Temps de réaction | Rendement en composé 1 | Pureté |
|---|---|---|---|---|
| 1 | Ethanol/acide acétique (5/1) | 10h | <10% non isolé évaluation CCM | Présence de matière 1^{ère}, d'intermédiaire réactionnel, de produits de dégradation |
| 2 | Acide acétique glacial pur à 80°C | 2h | 93% | > 95% |
| 3 | Ethanol/acide chlorhydrique 1N | 9h | 90% | 95% |
| 4 | Ethanol/eau/acide acétique (5/1/1) | 10h | 60% | 95% |
| 5 | Solvant : dioxane ou DMA/acide acétique/eau (5/1/1) | 5-6h | 70% | 95% |

Le tableau 1 montre l'avantage important de l'utilisation de l'acide acétique glacial pur à 80°C avec un temps de réaction court de 2h pour fournir le produit recherché avec un excellent rendement dans un état de pureté très satisfaisant pour une utilisation ultérieure en synthèse de composés anticancéreux.

## Revendications

1. Procédé de synthèse de la 4β-amino-4'-déméthyl-4-desoxypodophyllotoxine de formule 1 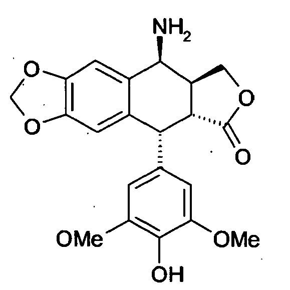 **caractérisé en ce qu'**il comprend les étapes successives suivantes :
a) réaction, dans un acide faible pur, sans autre solvant, à une température supérieure à la température ambiante, de la thiourée avec la 4β-halogénoacétamido-4'-déméthyl-4-desoxypodophyllotoxine de formule 3 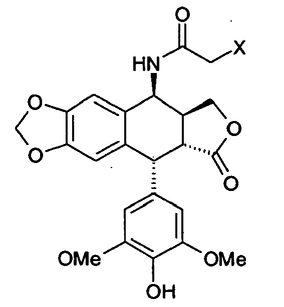 dans laquelle X représente un atome d'halogène choisi dans le groupe constitué par le chlore, le brome et l'iode, avantageusement le chlore ;
b) récupération de la 4β-amino-4'-déméthyl-4-desoxypodophyllotoxine.

2. Procédé selon la revendication 1, **caractérisé en ce que** l'acide faible pur est un acide carboxylique de formule 5 R-COOH, dans laquelle R représente un hydrogène ou un radical alkyle en C₁-C₂, en particulier l'acide acétique.

3. Procédé selon la revendication 1 ou 2, **caractérisé en ce que**, lors de l'étape a), le milieu réactionnel est chauffé à une température comprise entre 60 et 100°C.

4. Procédé selon l'une quelconque des revendications précédentes, **caractérisé en ce que**, lors de l'étape a), la 4β-halogénoacétamido-4'-déméthyl-4-desoxypodophyllotoxine est mise en contact avec l'acide faible pur avant ajout de la thiourée.

5. Procédé selon l'une quelconque des revendications précédentes, **caractérisé en ce que** le temps de réaction de l'étape a) est compris entre 1 et 3 heures.

6. Procédé de synthèse de la 4β-amino-4'-déméthyl-4-desoxypodophyllotoxine de formule 1 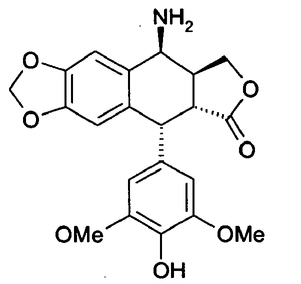 **caractérisé en ce qu'**il comprend les étapes successives suivantes :
i) réaction, dans un mélange d'acide, d'eau et de solvant organique, à une température supérieure à la température ambiante, de la thiourée avec la 4β-halogénoacétamido-4'-déméthyl-4-desoxypodophyllotoxine de formule 3 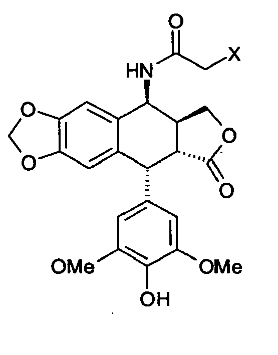 dans laquelle X représente un atome d'halogène choisi dans le groupe constitué par le chlore, le brome et l'iode, avantageusement le chlore ;
ii) récupération de la 4β-amino-4'-déméthylépipodophyllotoxine.

7. Procédé selon la revendication 6, **caractérisé en ce que**, lors de l'étape i), le milieu réactionnel est chauffé à une température comprise entre 60 et 100°C.

8. Procédé selon la revendication 6 ou 7, **caractérisé en ce que**, lors de l'étape i), la 4β-halogénoacétamido-4'-déméthyl-4-desoxypodophyllotoxine est mise en contact avec le mélange d'acide, d'eau et de solvant organique avant ajout de la thiourée.

9. Procédé selon l'une quelconque des revendications 6 à 8, **caractérisé en ce que** le solvant organique est un solvant organique hydrosoluble, avantageusement choisi dans le groupe constitué par les éthers cycliques, en particulier le dioxane, les alcools, en particulier l'éthanol, et le N,N-diméthylacétamide, le diméthylformamide, la N-méthylpyrrolidone.

10. Procédé selon la revendication 9, **caractérisé en ce que** le solvant est l'éthanol.

11. Procédé selon la revendication 10, **caractérisé en ce que** l'acide est un acide fort, en particulier choisi dans le groupe constitué par l'acide chlorhydrique, l'acide sulfurique et l'acide phosphorique.

12. Procédé selon l'une quelconque des revendication 6 à 10, **caractérisé en ce que** l'acide est un acide faible, en particulier un acide carboxylique de formule 5 R-COOH, dans laquelle R représente un hydrogène, un radical alkyle en C₁-C₂, de préférence l'acide acétique.

13. Procédé selon la revendication 12, **caractérisé en ce que** le rapport volumétrique éthanol ou dioxane ou N,N-diméthylacétamide, diméthylformamide, N-méthylpyrrolidone / eau / acide acétique est de 5/1/1.

14. Procédé selon l'une quelconque des revendications précédentes, **caractérisé en ce que** le rapport molaire entre la 4β-halogénoacétamido-4'-déméthyl-4-desoxypodophyllotoxine et la thiourée est compris entre 0,5 et 1.

15. Procédé selon l'une quelconque des revendications précédentes, **caractérisé en ce que** la 4β-halogénoacétamido-4'-déméthyl-4-desoxypodophyllotoxine de formule 3 est obtenue par réaction de la 4'-déméthylépipodophyllotoxine de formule 2 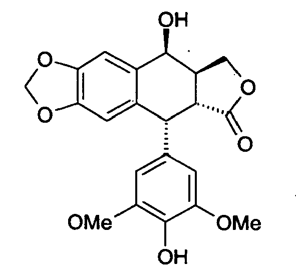 avec un halogénoacétonitrile de formule 6 X-CH₂-C≡N, dans laquelle X représente un atome d'halogène choisi dans le groupe constitué par le chlore, le brome et l'iode, en milieu acide.

## Claims

1. A method for synthesising 4β-amino-4'-demethyl-4-desoxypodophyllotoxin of formula 1 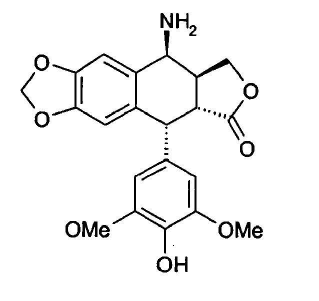 **characterised in that** it comprises the following successive stages:
a) reaction, in a pure weak acid, without another solvent, at a temperature greater than ambient temperature of thiourea with 4β- halogenoacetamido-4'-demethyl-4-desoxypodophyllotoxin of formula 3 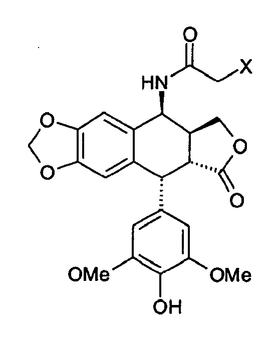 in which X represents a halogen atom selected from the group made up by chlorine, bromine and iodine, advantageously chlorine ;
b) recovery of 4β-amino-4'-demethyl-4-desoxypodophyllotoxin.

2. The method as claimed in Claim 1, **characterised in that** the pure weak acid is a carboxylic acid of formula 5 R-COOH, in which R represents hydrogen or an alkyl radical in C1-C2, in particular acetic acid.

3. The method as claimed in Claim 1 or 2, **characterised in that** during stage a) the reactional medium is heated to a temperature between 60 and 100°C.

4. The method as claimed in any one of the preceding claims, **characterised in that** during stage a) 4β-halogenoacetamido-4'-demethyl-4-desoxypodophyllotoxin is placed in contact with the pure weak acid prior to addition of thiourea.

5. The method as claimed in any one of the preceding claims, **characterised in that** the reaction time of stage a) is between 1 and 3 hours.

6. A method for synthesising 4β-amino-4'-demethyl-4-desoxypodophyllotoxin of formula 1 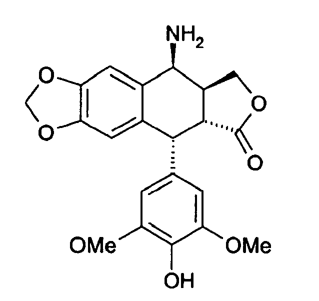 **characterised in that** it comprises the following successive stages:
i) reaction, in a mixture of acid, water and organic solvent, at a temperature greater than ambient temperature, thiourea with 4β- halogenoacetamido-4'-demethyl-4-desoxypodophyllotoxin of formula 3 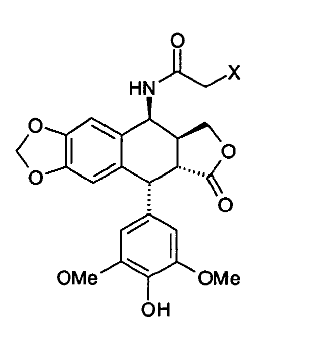 in which X represents a halogen atom selected from the group made up by chlorine, bromine and iodine, advantageously chlorine ;
ii) recovery of 4β-amino-4'-demethylepipodophyllotoxin.

7. The method as claimed in Claim 6, **characterised in that**, during stage i), the reactional medium is heated to a temperature between 60 and 100°C.

8. The method as claimed in Claim 6 or 7, **characterised in that**, during stage i), 4β-halogenoacetamido-4'-demethyl-4-desoxypodophyllotoxin is placed in contact with the mixture of acid, water and organic solvent prior to addition of thiourea.

9. The method as claimed in any one of Claims 6 to 8, **characterised in that** the organic solvent is a hydrosoluble organic solvent, advantageously selected from the group made up by cyclic ethers, in particular dioxane, alcohols, in particular ethanol, and N,N-dimethylacetamide, dimethylformamide, N-methylpyrrolidone.

10. The method as claimed in Claim 9, **characterised in that** the solvent is ethanol.

11. The method as claimed in Claim 10, **characterised in that** the acid is a strong acid, in particular selected from the group made up by hydrochloric acid, sulphuric acid and phosphoric acid.

12. The method as claimed in any one of Claims 6 to 10, **characterised in that** the acid is a weak acid, in particular a carboxylic acid of formula 5 R-COOH, in which R represents hydrogen, an alkyl radical in C1-C2, preferably the acetic acid.

13. The method as claimed in Claim 12, **characterised in that** the ethanol or dioxane or N,N-dimethylacetamide, dimethylformamide, N-methylpyrrolidone/water/acetic acid volumetric ratio is 5/1/1.

14. The method as claimed in any one of the preceding claims, **characterised in that** the molar ratio between 4β-halogenoacetamido-4'-demethyl-4-desoxypodophyllotoxin and thiourea is between 0.5 and 1.

15. The method as claimed in any one of the preceding claims, **characterised in that** the 4β-halogenoacetamido-4'-demethyl-4-desoxypodophyllotoxin of formula 3 is obtained by reaction of 4'-demethylepipodophyllotoxin of formula 2 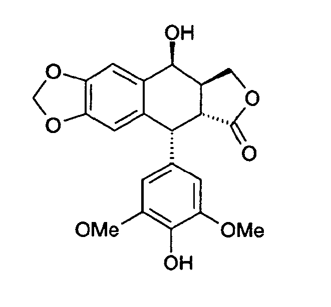 with a halogenoacetonitrile of formula 6 X-CH₂-C≡N, in which X represents a halogen atom selected from the group made up by the chlorine, the bromine and the iodine, in acid medium.

## Patentansprüche

1. Verfahren zur Synthese von 4β-Amino-4'-demethyl-4-desoxypodophyllotoxin der Formel 1 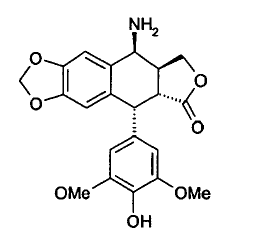 **dadurch gekennzeichnet, dass** es die folgenden aufeinanderfolgenden Schritte umfasst:
a) Reaktion in einer reinen schwachen Säure ohne anderes Lösungsmittel bei einer Temperatur über der Raumtemperatur von Thioharnstoff mit 4β-Halogenacetamid-4'-demethyl-4-desoxypodophyllotoxin der Formel 3 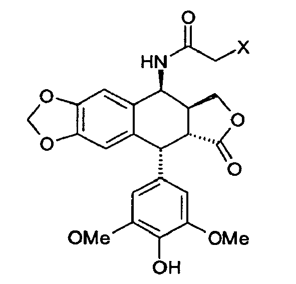 wobei X ein Halogenatom darstellt, das aus der Gruppe ausgewählt ist, die von Chlor, Brom und Jod gebildet wird, in vorteilhafter Weise von Chlor,
b) Rückgewinnung des 4β-Amino-4'-demethyl-4-desoxypodophyllotoxins.

2. Verfahren nach Anspruch 1, **dadurch gekennzeichnet, dass** die reine schwache Säure eine Carbonsäure der Formel 5 R-COOH ist, wobei R einen Wasserstoff oder ein C₁-C₂-Alkyradikal darstellt, insbesondere die Essigsäure.

3. Verfahren nach Anspruch 1 oder 2, **dadurch gekennzeichnet, dass** das Reaktionsmilieu in Schritt a) auf eine Temperatur zwischen 60 und 100 °C inklusive erhitzt wird.

4. Verfahren nach einem der vorangehenden Ansprüche, **dadurch gekennzeichnet, dass** das 4β-Halogenacetamid-4'-demethyl-4-desoxypodophyllotoxin in Schritt a) vor Hinzufügen des Thioharnstoffs mit der reinen schwachen Säure in Kontakt gebracht wird.

5. Verfahren nach einem der vorangehenden Ansprüche, **dadurch gekennzeichnet, dass** die Reaktionszeit von Schritt a) zwischen 1 und 3 Stunden inklusive beträgt.

6. Verfahren zur Synthese von 4β-Amino-4'-demethyl-4-desoxypodophyllotoxin der Formel 1 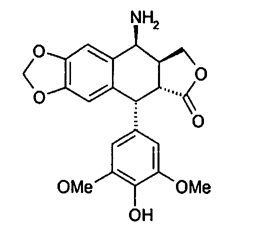 **dadurch gekennzeichnet, dass** es die folgenden aufeinanderfolgenden Schritte umfasst:
i) Reaktion in einem Gemisch aus Säure, Wasser und organischem Lösungsmittel bei einer Temperatur über der Raumtemperatur von Thioharnstoff mit 4β-Halogenacetamid-4'-demethyl-4-desoxypodophyllotoxin der Formel 3 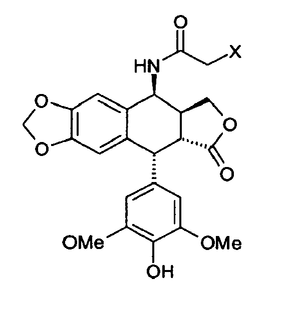 wobei X ein Halogenatom darstellt, das aus der Gruppe ausgewählt ist, die von Chlor, Brom und Jod gebildet wird, in vorteilhafter Weise von Chlor,
ii) Rückgewinnung des 4β-Amino-4'-demethylepipodophyllotoxins.

7. Verfahren nach Anspruch 6, **dadurch gekennzeichnet, dass** das Reaktionsmilieu in Schritt i) auf eine Temperatur zwischen 60 und 100 C inklusive erhitzt wird.

8. Verfahren nach Anspruch 6 oder 7, **dadurch gekennzeichnet, dass** das 4β-Halogenacetamid-4'-demethyl-4-desoxypodophyllotoxin in Schritt i) vor Hinzufügen des Thioharnstoffs mit dem Gemisch aus Säure, Wasser und organischem Lösungsmittel in Kontakt gebracht wird.

9. Verfahren nach einem der Ansprüche 6 bis 8, **dadurch gekennzeichnet, dass** das organische Lösungsmittel ein wasserlösliches organisches Lösungsmittel ist, das in vorteilhafter Weise aus der Gruppe ausgewählt ist, die von den zyklischen Ethern, insbesondere dem Dioxan, den Alkoholen, insbesondere dem Ethanol, und dem N,N-Dimethylacetamid, dem Dimethylformamid, dem N-Methylpyrrolidon gebildet wird.

10. Verfahren nach Anspruch 9, **dadurch gekennzeichnet, dass** das Lösungsmittel Ethanol ist.

11. Verfahren nach Anspruch 10, **dadurch gekennzeichnet, dass** die Säure eine starke Säure ist, insbesondere aus der Gruppe ausgewählt, die von der Salzsäure, der Schwefelsäure und der Phosphorsäure gebildet wird.

12. Verfahren nach einem der Ansprüche 6 bis 10, **dadurch gekennzeichnet, dass** die Säure eine schwache Säure ist, insbesondere eine Carbonsäure der Formel 5 R-COOH, wobei R einen Wasserstoff, ein C₁-C₂-Alkyradikal darstellt, vorzugsweise die Essigsäure.

13. Verfahren nach Anspruch 12, **dadurch gekennzeichnet, dass** das volumetrische Verhältnis Ethanol oder Dioxan oder N,N-Dimethylacetamid, Dimethylformamid, N-Methylpyrrolidon / Wasser / Essigsäure 5/1/1 beträgt.

14. Verfahren nach einem der vorangehenden Ansprüche, **dadurch gekennzeichnet, dass** das molare Verhältnis zwischen dem 4β-Halogenacetamid-4'-demethyl-4-desoxypodophyllotoxin und dem Thioharnstoff zwischen 0,5 und 1 inklusive ist.

15. Verfahren nach einem der vorangehenden Ansprüche, **dadurch gekennzeichnet, dass** das 4β-Halogenacetamid-4'-demethyl-4-desoxypodophyllotoxin der Formel 3 durch Reaktion des 4'-Demethylepipodophyllotoxins der Formel 2 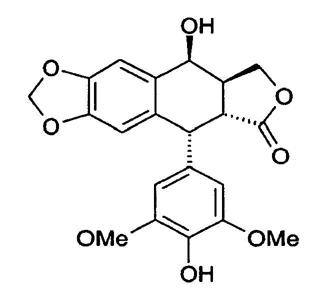 mit einem Halogenacetonitril der Formel 6 X-CH₂-C≡N hergestellt wird, wobei X ein Halogenatom darstellt, das aus der Gruppe ausgewählt ist, die von Chlor, Brom und Jod gebildet wird, in saurem Milieu.
